# EUROPEAN PATENT APPLICATION

(11) **EP 2 940 475 A1**
(43) Date of publication of application: **04.11.2015**
(21) Application number: 14305628.1
(22) Date of filing: 28.04.2014
(51) Int. Cl.: G01N 33/94

(54) **Hashish marker**

(71) Applicant: CENTRE NATIONAL DE LA RECHERCHE SCIENTIFIQUE -CNRS-, 75794 Paris Cedex 16 (FR); Universite De Nice-Sophia Antipolis, 06108 Nice Cedex 02 (FR)
(72) Inventor: Filippi, Jean-Jacques, 06100 NICE (FR); Marchini, Marie, 13500 MARTIGUES (FR); Baldovini, Nicolas, 06200 NICE (FR)
(74) Representative: Osha Liang

(57) **Abstract**

A method for detecting the presence of hashish in a sample, the method comprising: detecting a presence of 5,5-dimethyl-1-vinylbicyclo[2.1.1]hexane (1) in the sample.

## Description

### TECHNICAL FIELD

The present disclosure relates to a method and a device for detecting the presence of hashish in a sample. The present disclosure also relates to the use of 5,5-dimethyl-1-vinylbicyclo[2.1.1]hexane as marker of hashish in a sample.

### BACKGROUND

Among all drugs of abuse, cannabis remains one of the most consumed worldwide under all of its various forms (marijuana, hashish, solvent extract). The resin of *Cannabis sativa* L., known as hashish, is particularly popular in western countries owing to its low price compared to other common illegal drugs, and it is commonly considered a drug of lower addictive impact while discussions about bringing it to legality are still ongoing in some European countries. Hashish is a product of interest for drug dealers because of the malleability of such a resin, which allows easy concealment and transportation. Hashish is produced in various countries around the world. Hashish is produced via a relatively long process that consists in successively drying and sieving the resin-rich buds of cannabis plants that have been harvested at full blooming stage. Hashish is generally a medium hard greenish-brownish paste that softens upon heating. The demand for hashish is still very high in Europe making hashish illegal market a major concern for European authorities, requiring more and more means for efficient traffic control.

Research of illegal drugs, made by customs squads at borders or during police controls, has usually been requiring canine assistance for detection of target substances. The use of trained dogs is an efficient mean for rapid drug detection. Also, trained dogs are efficient in terms of detection sensitivity. However, the use of trained dogs has a certain drawback since a dog provides a working time limited to a few hours a day. Moreover, the high cost of a canine team is also a reason why people have long sought to replace, or rather say, complement detection dogs by the use of electronic devices designed for the specific detection of illicit substances such as drugs and explosives. In addition, detection by dogs is not recognized as a formal legal proof.

In relation with its psychotropic activity, *Cannabis sativa* L. has been mainly studied for the determination of its content in cannabinoids. A large variety of molecules has been reported, the most representative being cannabidiol (CBD), cannabinol (CBN), and Δ⁹-tetrahydrocannabinol (Δ⁹-THC) which is known as the main psychoactive principle of the drug. In comparison with cannabinoid derivatives, the volatile constituents of cannabis drugs have received much less attention. Nevertheless, the chemical composition of the volatile fraction of *Cannabis sativa* L. derived products is well documented and has been mainly studied via the analysis of their essential oils or by means of headspace sampling techniques (see, for example, Bertoli A, Tozzi S, Pistelli L, Angelini LG. Fibre hemp inflorescences: From crop-residues to essential oil production. Ind Crop Prod. 2010;32:329-37). A large variety of volatile organic compounds representing all chemical class (hydrocarbons, alcohols, ketones, esters) has been detected in *Cannabis sativa* L. samples, and efforts have been paid to establish a chemical profile of each form of cannabis drug and eventually determine a more or less steady odor that may be used as a chemical basis for detection of cannabis drug in the frame of criminal investigations (see, for example, Ross SA, ElSohly MA. The Volatile Oil Composition of Fresh and Air-Dried Buds of Cannabis sativa L. J Nat Prod. 1996;59:49-51). All previous studies have reported a high content of monoterpenes and sesquiterpenes. In the monoterpene family, the most cited constituent are α- and β-pinene, β-myrcene, and limonene, while β-caryophyllene and α-humulene are predominant among sesquiterpenes. A quick overview of the main terpenoids reported as volatile constituents of *Cannabis sativa* L. is given in Table 1.

**Table 1. Most cited terpenoids previously identified among the volatile fraction of Cannabis sativa L. samples (Retention Indices (RI) from the literature; RI mainly taken from the Terpenoids Library (Mass Finder 4.0) available online at http://massfinder.com/wiki/Terpenoids_Library_List).**

| **Compound name** | **RI** | **Compound name** | **RI** |
|---|---|---|---|
| *Monoterpenes* | | α-Copaene | 1379 |
| Tricyclene | 927 | β-Elemene | 1389 |
| α-Thujene | 932 | Isocaryophyllene (syn. *cis-*caryophyllene) | 1409 |
| α-Pinene | 936 | | |
| Camphene | 950 | Longifolene | 1411 |
| Sabinene | 973 | *cis*-α-Bergamotene | 1412 |
| β-Pinene | 978 | (*E*)-β-Caryophyllene | 1421 |
| β-Myrcene | 987 | *trans*-α-Bergamotene | 1434 |
| α-Phellandrene | 1002 | α-Guaiene | 1440 |
| Δ³-Carene | 1010 | (*E*)-β-Farnesene | 1446 |
| α-Terpinene | 1015 | α-Humulene | 1455 |
| *p*-Cymene | 1015 | *allo*-Aromadendrene | 1462 |
| β-Phellandrene | 1023 | γ-Muurolene | 1474 |
| Limonene | 1025 | β-Selinene | 1486 |
| *cis*-β-Ocimene | 1029 | α-Selinene | 1494 |
| *trans*-β-Ocimene | 1041 | α-Curcumene | 1473 |
| γ-Terpinene | 1051 | Valencene | 1494 |
| α-Terpinolene | 1082 | β-Bisabolene | 1503 |
| | | γ-Cadinene | 1507 |
| *Monoterpenoids* | | δ-Cadinene | 1520 |
| *trans*-Sabinene hydrate | 1053 | Selina-4(14),7(11)-diene | 1534 |
| Fenchone | 1069 | Selina-3,7(11)-diene | 1542 |
| *cis*-Linalool oxide (furanoid) | 1072 | Germacrene-B | 1552 |
| Linalool | | | |
| Eucalyptol (1,8-cineole) | 1086 | *Sesquiterpenoids* | |
| Fenchol | 1024 | Nerolidol (*Z*: 1522; *E*: 1553) | |
| Ipsdienol | 1099 | Caryophyllene oxide | 1546 |
| Borneol | 1123 | Humulene oxide | 1593 |
| Terpinen-4-ol | 1150 | α-Guaiol | 1593 |
| α-Terpineol | 1264 | γ-Eudesmol | 1618 |
| | 1276 | β-Eudesmol | 1641 |
| *Sesquiterpenes* | | α-Eudesmol | 1653 |
| α-Cubebene | | β-Bisabolol | 1659 |
| α-Ylangene | 1355 | α-Bisabolol | 1673 |
| | 1376 | epi-α-Bisabolol | |

However, monoterpenes and sesquiterpenes reported in high content are not specific markers of cannabis drugs. For example, α-pinene, β-pinene, β-myrcene, limonene, β-caryophyllene and α-humulene are constituents of many essential oils. Also, the chromatographic profile of any cannabis drug sample generally depends on the nature of the cannabis drug and the method applied for the extraction of volatile organic compounds (VOCs) from the cannabis drug. For example, from the analysis of *Cannabis sativa* L. essential oil, it has been previously reported that fresh buds of *Cannabis sativa* L. show a chemical composition that is qualitatively similar to that of air-dried buds, but that the drying process results in a loss of the most volatile compounds, particularly affecting the content in monoterpenes. Moreover, the direct comparison of the chemical composition of an essential oil of cannabis with the chemical composition of a headspace sample is not often straightforward since thermolabile or water-sensitive constituents might be affected by hydrodistillation.

Accordingly, there exists a continuing need to provide markers, methods and devices for the specific detection of cannabis drugs such as hashish.

### SUMMARY

An object of the present disclosure is to provide a new marker as well as improved methods and devices adapted to detect cannabis drugs.

According to a first aspect, the above-mentioned objects, as well as further advantages, are achieved by a method for detecting the presence of hashish in a sample, the method comprising: detecting a presence of 5,5-dimethyl-1-vinylbicyclo[2.1.1]hexane in the sample.

According to a second aspect, one or more of the above-mentioned objects may be achieved by the use of 5,5-dimethyl-1-vinylbicyclo[2.1.1]hexane as marker of the presence of hashish in a sample.

According to a third aspect, one or more of the above-mentioned objects may be achieved by a device for detecting the presence of hashish in a sample, the device comprising: means for detecting a presence of 5,5-dimethyl-1-vinylbicyclo[2.1.1]hexane in the sample.

Other aspects and advantages of the present disclosure will be apparent from the following Figures, description and appended claims.

### BRIEF DESCRIPTION OF DRAWINGS

The present invention will be better understood and other advantages and embodiments will become clear on reading the description that follows, given purely by way of indication and in no way limiting, and by referring to the appended Figures in which:
Figure 1 shows VOCs composition analyses of fresh cannabis buds compared to a hashish sample detected according to an embodiment of the method of the present disclosure.
Figure 2 shows VOCs composition analyses of fresh (B) and dry cannabis buds (C) compared to a hashish sample (A) detected according to an embodiment of the method of the present disclosure.
Figure 3 shows the structure and EI-mass spectrum of 5,5-dimethyl-1-vinylbicyclo[2.1.1]hexane.
Figure 4 shows a VOCs composition analysis of a hashish sample detected according to an embodiment of the method according of the present disclosure.
Figure 5 shows VOCs composition analyses of cannabis buds compared to hashish samples detected according to an embodiment of the method according of the present disclosure.
Figures 6a, 6b, 6c and 6d show VOCs compositions of cannabis buds and hashish samples detected according to an embodiment of the method according of the present disclosure.

### DETAILED DESCRIPTION OF EMBODIMENTS

Embodiments of the present disclosure will now be described in detail with reference to the accompanying Figures. In the following detailed description of embodiments of the present disclosure, numerous specific details are set forth in order to provide a more thorough understanding of the present disclosure. However, it will be apparent to one of ordinary skilled in the art that the present disclosure may be practiced without these specific details. In other instances, well-known features have not been described in detail to avoid unnecessarily complicating the description.

Herein, the words "comprise/comprising" are synonymous with (means the same thing as) "include/including," "contain/containing", are inclusive or open-ended and do not exclude additional, non-recited elements. Further, herein the term "about" and "substantially" are synonymous with (means the same thing as) a 20 % margin of the respective value.

In the following, it is meant by "volatile organic compound" and "volatile component" organic compound having a high vapor pressure, such as greater than 0.01 kPa at 20 °C.

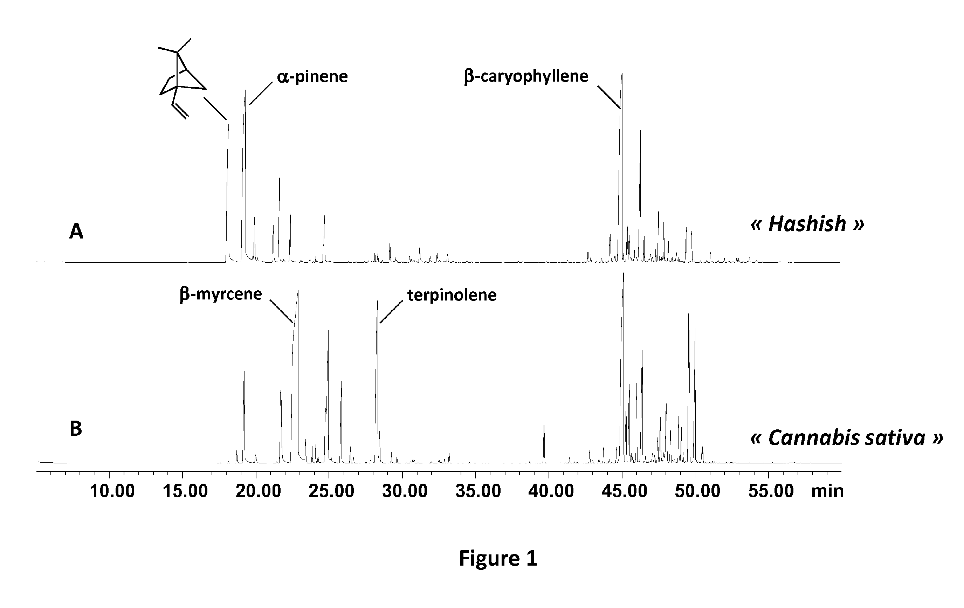

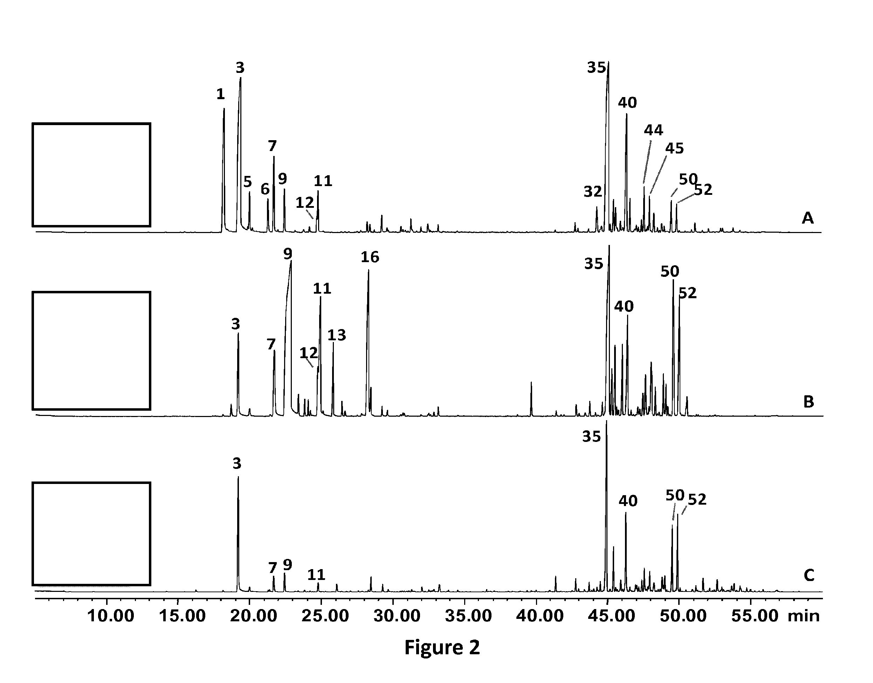

In order to provide a method, as well as a marker and a device, capable of detecting cannabis drugs, such as hashish, for example via VOCs emitted by the cannabis drugs, the Applicants have found that, among the different forms of cannabis drugs investigated, samples of *Cannabis sativa* L. resins (*i*.*e*. hashish) comprise 5,5-Dimethyl-1-vinylbicyclo[2.1.1]hexane (*i*.*e*. compound 1), which is a very rare monoterpene hydrocarbon. Indeed, compound 1 was found to be particularly abundant in hashish samples (e.g. samples A of Figures 1 and 2) while being either absent or present in lower amounts (e.g. lower than 1 % in the headspace) in other products such as fresh cannabis buds (e.g. sample B of Figures 1 and 2) and dried cannabis buds (e.g. sample C of Figure 2). For example, analytical methods such as GC-MS performed on a series of different samples of cannabis drugs (hashish and herb) allowed the Applicants to detect and identify compound 1 as being among the most abundant VOCs of hashish samples (see Figures 1, 2 and 3). In contrast, cannabis buds (*i*.*e*. herb) were found to contain compound 1 in lower amount.

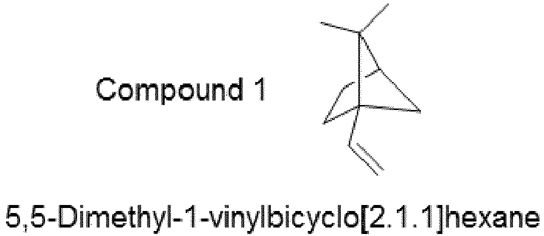

In view of the above, according to a first aspect, the present disclosure provides a method for detecting the presence of hashish in a sample, the method comprising: detecting a presence of 5,5-dimethyl-1-vinylbicyclo[2.1.1]hexane in the sample. In one or more embodiments, the method according to the present disclosure may comprise providing the sample, separating constituents from the sample, subjecting the constituents to an analytical method, and identifying 5,5-dimethyl-1-vinylbicyclo[2.1.1]hexane among the constituents. For example, in one or more embodiments, the constituents may comprise volatile organic compounds such as monoterpenes. In one or more embodiments, 5,5-dimethyl-1-vinylbicyclo[2.1.1]hexane may be identified among the monoterpenes. For example, the presence of hashish in the sample may be detected if 5,5-dimethyl-1-vinylbicyclo[2.1.1]hexane (1) amounts for at least 0.02 weight percent of the sample.

The natural occurrence of compound 1 in a natural environment, such as in plants, is generally very limited. For example, compound 1 has only been cited as a minor constituent of the essential oil of *Mentha cardiaca* (<0.1 %) (see, Hogg JW, Lawrence BM. Essential oils and their constituents. VIII. New bicyclic monoterpene from Mentha x cardiaca. Flavour Ind. 1972;3:321-3). In fact, the literature related to compound 1 is very scarce since only few references could be found as registered in the Chemical Abstract service (SciFinder), dealing generally with the synthetic preparation of compound 1 (see, for example, Liu RSH, Hammond GS. Mechanisms of photochemical reactions in solution. XXVII. Photosensitized cyclization of myrcene. Problem of addition of dienes to alkenes. J Am Chem Soc. 1964;86:1892-3). More specifically, compound 1 has been described as a photolytic product of β-myrcene (compound 9), through UV light-mediated rearrangement when irradiated in presence of sensitizers such as 2-acetylnaphtalene, benzophenone, or clay-supported materials.

Conversely, the literature related to *Cannabis sativa* L. (*e*.*g*. essential oil, VOCs in the Headspace) does not contain any mention of compound 1 among the identified VOCs. In contrast, other monoterpenes (α-pinene 3, β-pinene 7, limonene 11) and sesquiterpenes (β-caryophyllene 35 and α-humulene 40) are frequently reported as abundant. Among them, compound 9 has often been cited as a major volatile constituent and had thus been proposed as a specific marker of cannabis drugs such as in the form of hashish and marijuana.

Without being bound to any particular theory, the Applicants deem that the presence of compound 1 in hashish samples may be related to the high abundance of β-myrcene (compound 9) in the fresh herb of *Cannabis sativa* L.. Indeed, hashish is known to be generally manufactured from fresh herb according to a lengthy process involving several steps of sieving and drying of the resin-rich female heads of cannabis plants until the resin naturally aggregates to form hashish after a final compression. Sun exposure during drying steps is thus assumed to produce photolytic formation of compound 1 from compound 9. Indeed, any exposure to sunlight during those drying steps may be considered as responsible for the photolytic formation of compound 1 from compound 9.

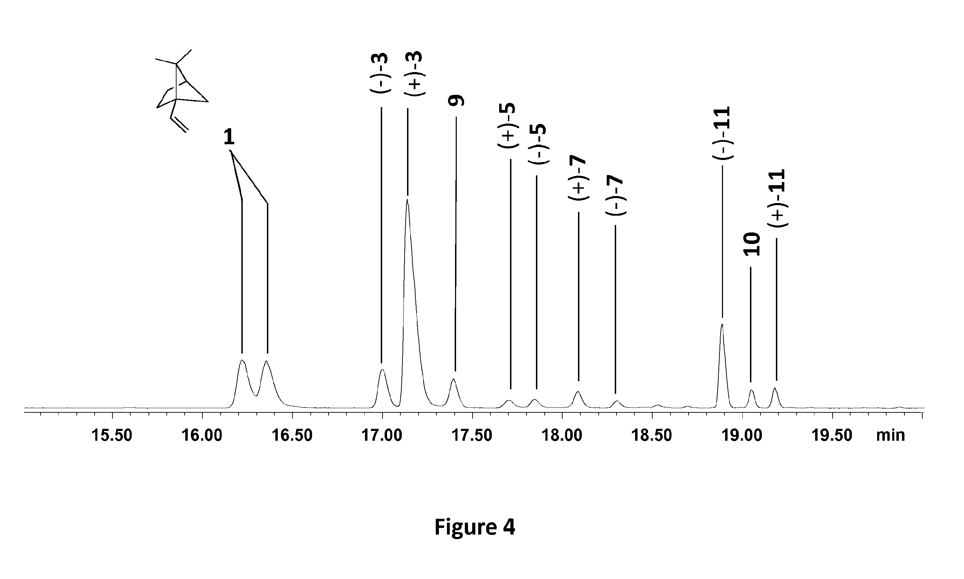

In one or more embodiments, the analytical method may comprise an enantioselective analytical technique. For example, the enantioselective analytical technique may be an enantioselective Gas Chromatography (GC) or High Performance Liquid Chromatography (HPLC) optionally coupled to or performed in combination with at least one additional analytical method selected from the group comprising mass spectrometry (MS), infrared (IR), RAMAN, UV/UV-visible spectroscopies, nuclear magnetic resonance (NMR), and ion-mobility spectrometry (IMS). In one or more embodiments, compound 1 may be identified as a racemate. For example, compound 1 may be in the form of a racemic mixture of a first enantiomer (1R,4S)-5,5-dimethyl-1-vinylbicyclo[2.1.1]hexane and of a second enantiomer (1S,4R)-5,5-dimethyl-1-vinylbicyclo[2.1.1]hexane, the amounts of both first and second enantiomers being substantially equals (e.g. 50 mol. % ± 5). Indeed, compound 1 may be a racemic photolytic product of compound 9. More specifically, the Applicants were able to determine that compound 1 was present as a racemic mixture in hashish by means of enantioselective analytical techniques such as enantioselective GC-MS (Figure 4), which further indicates that compound 1 may be of photolytic origin. Conversely, an enzymatic formation would have more reasonably induced some enantiomeric excess in compound 1. Figure 4 shows an enantioselective GC-MS analysis of monoterpenes from an hashish sample on heptakis(2,3-di-O-methyl-6-O-tert-butyldimethylsilyl)-β-cyclodextrin (Elution order is deduced from reference substances or literature data).

In one or more embodiments, separating constituents from the sample may be based on a technique selected from the group comprising gas chromatography, liquid chromatography, sorbent extraction, (VOC) trapping, solvent extraction (e.g. Soxhlet extraction), and any combination thereof. For example, a trapping module may comprise molecular captands and/or sensors. In one or more embodiments, separating constituents from the sample may comprise headspace (HS) sampling. Indeed, HS sampling appears to be a least invasive analytical way to investigate cannabis drug samples, such as hashish samples. For example, HS sampling may provide compound 1 in an amount sufficient for detection. Also, HS sampling may provide representative profiles of VOCs present in the samples.

In one or more embodiments, the HS sampling may be a direct HS sampling. Indeed, direct HS sampling may be use to provide good representative profile of the VOCs emitted by a sample. For example, direct HS sampling may comprise the use of a trapping module such as a headspace autosampler, a large volume injector, and/or programmed temperature vaporizer (PTV).

In one or more embodiments, separating (e.g. extracting) constituents from the sample may comprise solid phase microextraction (SPME) such as SPME in headspace mode (HS-SPME) or in immersion mode. Indeed, SPME may provide sampling of smaller volumes, for example with respect to direct HS sampling. In one or more embodiments, the SPME may comprise a preconcentration step of the VOCs comprised in the sample. In this way, SPME may provide further sensitivity to the method according to the present disclosure. Also, SPME may be easier to implement to set up. For example, SPME may be run on any GC(-MS) system with no or minimum modifications.

In one or more embodiments, SPME may be performed from about 10°C to about 70°C. For example, SPME may be performed from about 15°C to about 30°C and preferably at about room temperature (22°C ± 5). In one or more embodiments, SPME may be performed for about 10 min to about 1 hour. For example, SPME may be performed from about 15 min to about 45 min and preferably for about 20 min. In one or more embodiments, the sample may comprise at least 0.1 g of hashish. For example the sample may comprise at least 0.25 g of hashish and preferably about 1 g. In one or more embodiments, the preconcentration may be performed for about 10 min to about 2 hour. For example, the preconcentration may be performed from about 15 min to about 1 hour and preferably for about 30 min.

In one or more embodiments, HS sampling may be performed with solid phase microextraction (SPME) kits such as SPME fibers. For example, SPME may be performed using a polydimethylsiloxane (PDMS) SPME fiber such as a 100 µm polydimethylsiloxane (PDMS) SPME fiber. However, HS sampling may be carried using other techniques. For example, in one or more embodiments, HS sampling may performed using an equipment selected from the group comprising gas-tight syringes, a SPME kits, tenax® adsorbents (*e*.*g*. porous polymeric resin comprising 2.6-diphenylene oxide), and a trapping tool. For example, the trapping tool may comprise molecular captands and/or sensors.

In one or more embodiments, compound 1 may amount for at least 0.02 weight %, preferably at least 0.1 weight %, and more preferably at least 0.2 weight % of the sample. So for example, using a method comprising HS sampling, the compound 1 may amount for at least 1 %, preferably at least 5 %, and more preferably at least 10 % of the sample in the headspace.

In one or more embodiments, the analytical method may be either qualitative or quantitative. In one or more embodiments, the analytical method is quantitative. In one or more embodiments, the analytical method may be selected from the group comprising a chromatography technique (*e*.*g*. GC, LC, HPLC), mass spectrometry (MS), infrared (IR), RAMAN, UV/UV-visible spectroscopies, nuclear magnetic resonance (NMR), ion-mobility spectrometry (IMS), and any combination thereof. In one or more embodiments, the chromatographic technique may be based on gas chromatography (GC). In one or more embodiments, the analytical method may comprise a gas chromatograph (GC) coupled to or used in combination with a mass spectrometer (MS), and/or an infrared (IR) spectrometer, and/or an ion-mobility spectrometer (IMS), and any combination thereof.

A second aspect of the present disclosure is to provide a use of 5,5-dimethyl-1-vinylbicyclo[2.1.1]hexane as marker of the presence of hashish in a sample. Indeed, compound 1 is very rare among natural constituents of essential oils. For example, merely a scarce number of references relating to compound 1 is found in the prior art. Further, a single reference deals with the identification of compound 1 in a natural extract in trace levels.

A third aspect of the present disclosure is to provide a device, such as an apparatus, for detecting the presence of hashish in a sample, the device comprising: means, such as a device, for detecting the presence of compound 1 in the sample. For example, the device for detecting the presence of hashish in a sample may be configured to detect the presence of compound 1 in the sample.

In one or more embodiments, the means for separating compound 1 from the sample may comprise one selected from the group comprising a gas chromatography system, a liquid chromatography system, a sorbent extraction tool, a trapping module, a solvent extractor and any combination thereof. In one or more embodiments, the sorbent extraction tool may comprise a solid phase microextraction (SPME) kit, a gas-tight syringe, and/or a tenax® adsorbent. In one or more embodiments, the trapping module may comprise molecular captands and/or sensors.

In one or more embodiments, the means for identifying may comprise a system selected from the group comprising gas chromatography (GC), liquid chromatography (LC), mass spectrometry (MS), infrared (IR), RAMAN, and UV/UV-visible spectroscopies, nuclear magnetic resonance (NMR), ion-mobility spectrometry (IMS), and any combination thereof. In one or more embodiments, the means for identifying may comprise a gas chromatograph (GC) coupled to a mass spectrometer (MS), and/or an infrared (IR) spectrometer, and/or an ion-mobility spectrometer (IMS), and any combination thereof.

For example, crossing data from an IR spectrometer and an IMS may allow detection of compound 1. Thus, devices (and methods) according to the present disclosure comprising (using) both infrared and ion-mobility spectrometers may be used, for example by customs, to detect cannabis drugs such as hashish, for example in containers or trucks.

In the same manner, devices and methods according to the present disclosure may be based on miniaturized GC (*e*.*g*. miniature ion trap mass spectrometer) and/or miniaturized MS techniques and apparatuses. For example, the device may be a portable detector (*e*.*g*. the size of a suitcase). Portable detectors are ideal for customs or border police, which may thus detect compound 1 and conclude the presence of cannabis drugs such as hashish, for example, in a container or a truck. Also devices according to the present disclosure may be fixed detectors (*i*.*e*. not portable), for example for airports, public places, and the like.

### EXAMPLES

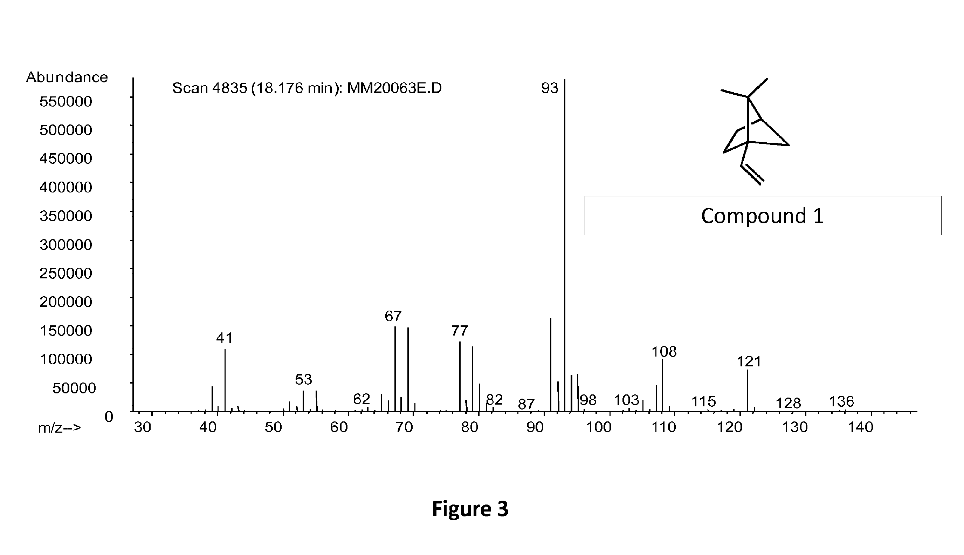

The Applicants show here exemplary analytical methods and results, which allow detection of compound 1, which is identified as a marker, for example as VOC marker, of the presence of hashish in a sample. Different forms of cannabis drugs (fresh and dried buds, and hashish) have been subjected to analytical method, for example by gas chromatography (GC), mass spectrometry (MS), and/or headspace sampling techniques. As shown in Figure 3, compound 1 was identified on the basis of the mass spectrum of compound 1, which is available in a commercial MS databank (MassFinder 4.0, Germany), and IR properties of compound 1.

Chemicals and products: Samples of resins (hashish) and buds of *Cannabis sativa* L. were obtained from the Institut National de Police Scientifique (INPS). All chemicals and solvents were purchased from Sigma-Aldrich and used without further purification otherwise stated.

Exemplary solvent extraction: 15 g of hashish (probably originating from Morocco) were extracted in a 200 mL Soxhlet apparatus with 250 mL of pentane for 5 hours. The resulting extract (∼10 g) mainly contained cannabinoids and was subsequently chromatographed on silica gel with eluents of increasing polarity. The first fraction (1.2 g), eluted with pentane, contained mostly monoterpenes and sesquiterpenes, accompanied with a limited series of diterpenes. In this fraction, compound 1 was determined to amount for ∼4.5 % by GC-MS analysis.

Exemplary SPME: 100 % Polydimethysiloxane (PDMS; 100 µm) and Divinylbenzene/Carboxen/Polydimethyl-siloxane (DVB/CAR/PDMS; 50/30 µm) SPME fibers were purchased from Supelco Europe. Improved parameters for SPME analyses (incubation/extraction-time/temperature, nature of the fiber coating) may be selected following a methodology described by Vanot GE, Sergent M. Experimental Design in Microbiology. In: Barredo J-L, editor. Microbial Processes and Products: Humana Press; 2005. p. 25-39.

Gas chromatography-mass spectrometry (GC-MS): GC-MS analyses were run on two GC-MS systems. For SPME analyses, gas chromatographs were operated in splitless mode with a constant flow (1 mL/min) of Helium. For other routine analyses, GC systems were operated in split mode (1/50). At Université Nice Sophia-Antipolis (UNS), an Agilent 6890N/5973N system equipped with either a J&W HP-1 capillary column (50 m × 0.2 mm; 0.33µm film thick.) was used. Oven temperature program: initial temperature, 50°C, then increased to 250°C with a constant rate of 2°C/min. At INPS, an Agilent 7890A/5975C system was mounted with a Varian VF-1MS capillary column (50 m × 0.25 mm; 0.25 µm film thick.). The oven was programmed as follows: initial temperature, 60°C, then increased to 250°C with a constant rate of 3°C/min, transfer-line temperature: 280°C. For both systems, the transfer-line temperature was set at 280°C. Electron impact mass spectra were acquired over a 35-350 m/z range. Volatile constituents were identified upon cross-correlation of their retention indices (RI) calculated from a series of *n*-alkanes, and their mass spectra matched against commercial librairies (Wiley275, NIST08, MassFinder 2.1) or in-house MS databases built from literature information and preferably against isolated or synthesized substances whenever available.

Enantioselective gas chromatography: Separation of the two enantiomers of 5,5-dimethyl-1-vinylbicyclo[2.1.1]hexane was carried out using a Macherey-Nagel Hydrodex β-6TBDM capillary column mounted on a Agilent 6890N/5973N GC-MS system. Split ratio 1/100; Oven temperature program: 80°C to 220°C at 2°C/min. Constant flow of helium: 1.0 mL/min. MS parameters were used as described above for SPME experiments.

UV-mediated synthesis of 5,5-dimethyl-1-vinyl bicyclo[2.1.1]hexane: Compound 1 was synthesized according to a protocol inspired from Liu and Hammond (reference herein above). A mixture of compound 9 (1.63 g; 12 mmol) and 2-acetylnaphtalene (23 mg) was dissolved in 70 mL of dry diethylether and placed in a UV lamp reactor system (Heraeus, Germany). The mixture was irradiated under argon atmosphere until total consumption of compound 9 as monitored by GC analysis of aliquots withdrawn from the reaction mixture. The solvent was carefully removed under vacuum, and the crude product was obtained as nearly colorless oil (crude yield > 90 %; ∼67 % GC purity). The reaction was repeated several times and all crude products combined in order to increase the amount of material for distillation. A mixture of crude reaction products (12.5 g) was submitted to distillation under vacuum. Compound 1 (6.5 g) was isolated with 87 % purity (bp 50°C under 0.3 mbar).

Nuclear magnetic resonance: compound 1 was also identified by High resolution NMR spectra recorded in CDCl₃ at 298 K on a Bruker Avance DRX 500 spectrometer operating at 500.13 MHz for ¹H and 125.76 MHz for ¹³C. In order to increase sensitivity, ¹³C-NMR spectra such as broadband-¹³C and JMOD were run with a direct probe head (5 mm PADUL ¹³C-1H Z-GRD). 1 D- and 2D-NMR spectra such as 1 H, COSY, TOCSY, NOESY, HSQC, HMBC were run with an inverse probe head (5 mm PHTXI ¹H-¹³C/15N ZGRD). Spectrum calibration was performed by using CDCl₃ signal as internal reference (7.26 ppm for ¹H, 77.16 ppm for ¹³C). Chemical shifts (δ) were expressed in parts per million (ppm) and coupling constants (J) in hertz. All NMR experiments were carried out using pulse sequences supplied by the spectrometer manufacturer (Bruker Topspin^{™}) and processed via Mestrelab MestreNOVA software (v6.0.2-5475).

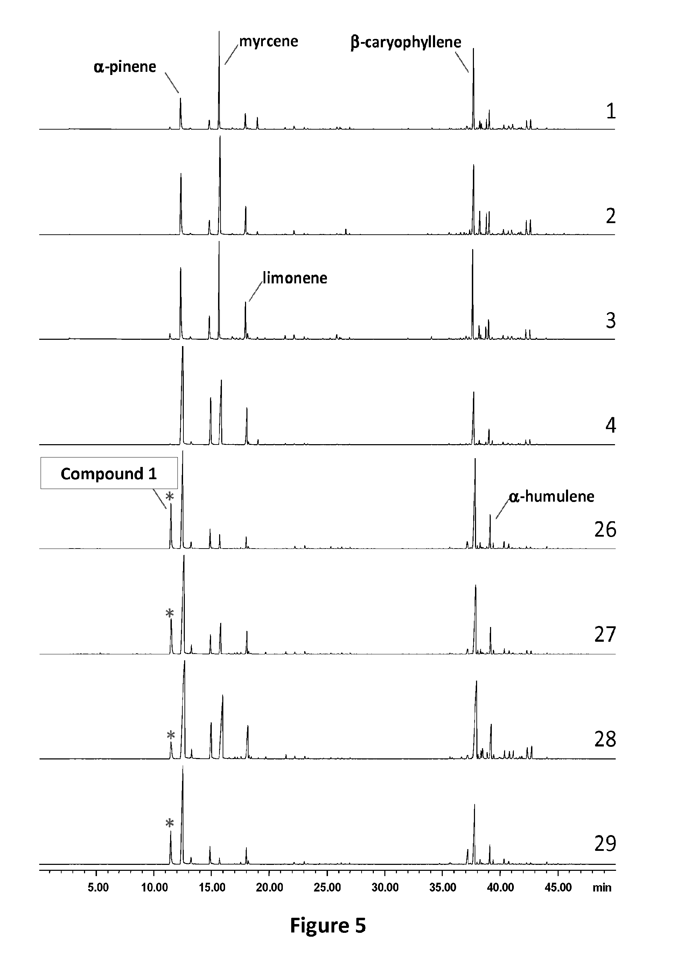

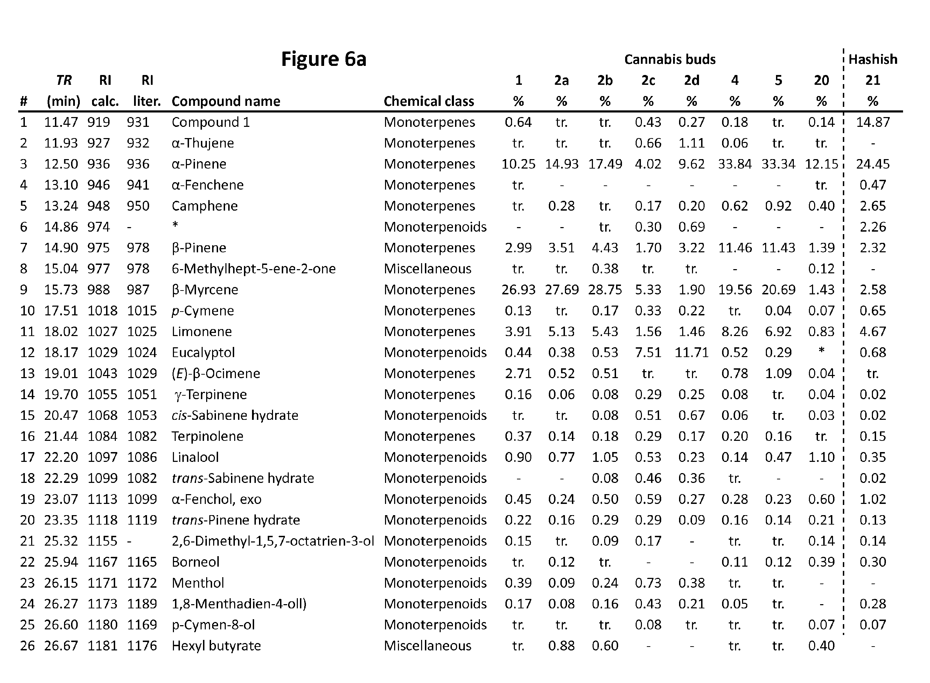

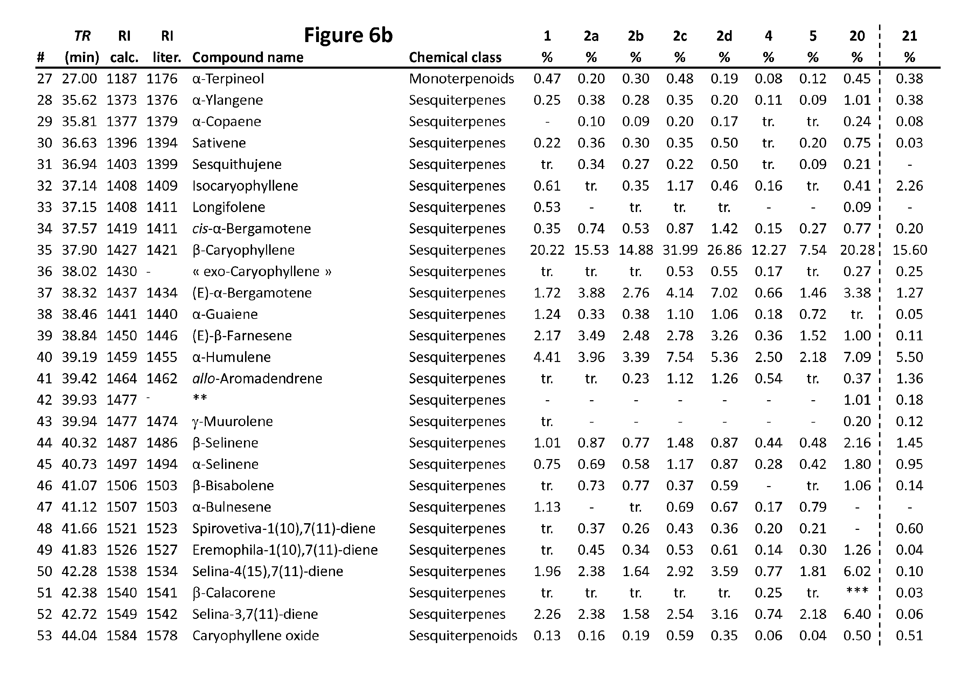

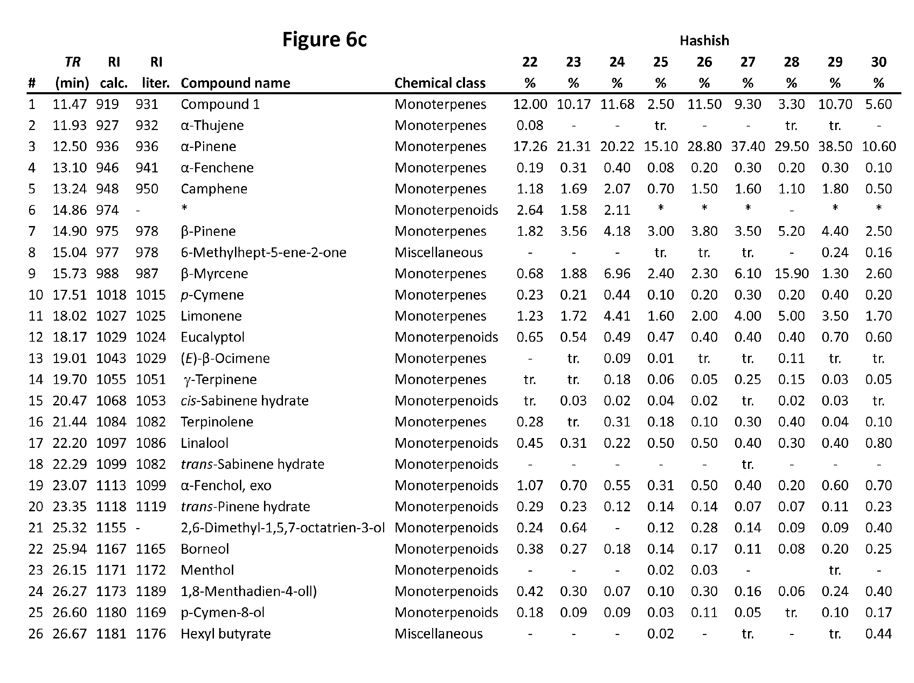

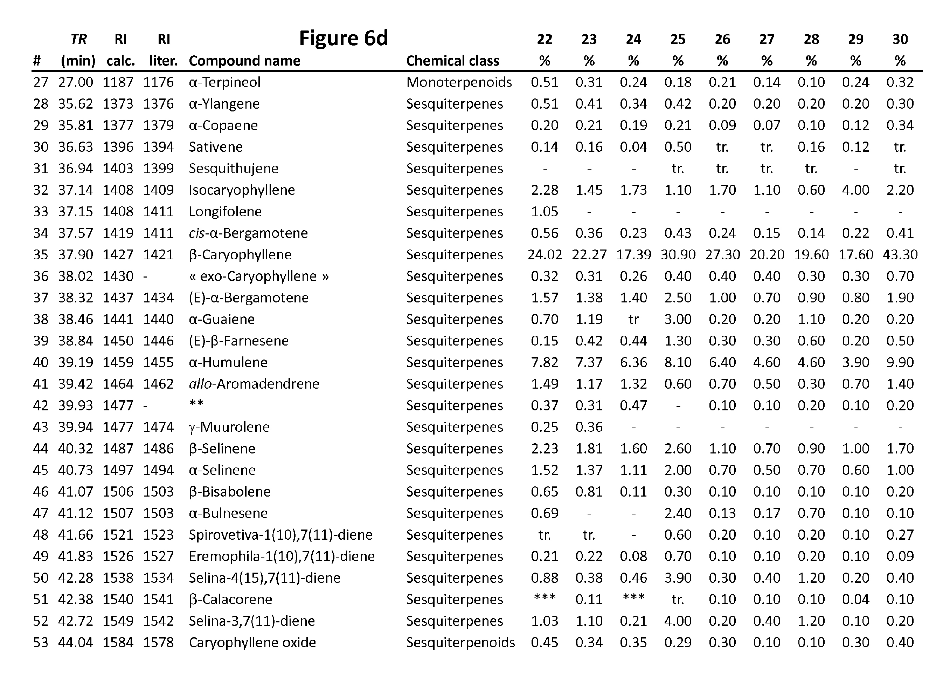

A series of analyses was performed on a higher number of samples. More specifically, 10 samples of hashish and 8 samples of cannabis buds (fresh and dried) were analyzed by means of HS-SPME-GC-MS. In hashish samples, compound 1 was detected among the most abundant compounds in the headspace, ranging from 2.5 to 14.9 %. Conversely, compound 1 was detected either at trace levels or in very low amounts in fresh and dried buds (see Figures 5 and 6a-d). Figure 5 shows Comparative HS-SPME-GCMS analyses of multiple samples of cannabis buds (1-4) and hashish (26-29) provided by the INPS. While several terpenes are commonly found in all forms of cannabis drugs, as shown in Figure 5, chromatographic profiles of hashish samples may be characterized by the presence of compound 1 eluting before α-pinene. In Figures 6a-d: RI calc. represents the retention indexes calculated against a series of C6-C24 n-alkanes; RI liter. represents the retention index from the terpenoids Library lists (available for example at massfinder.com); exo-Caryophyllene is 10,10-dimethyl-2,6-bis(methylene)bicyclo[7.2.0]undecane (CAS# 136296-38-3); Spirovetiva-1(10),7(11)-diene is often coeluted with other sesquiterpenes such as δ-cadinene; samples of cannabis buds 2a-d were indexed by INPS as coming from the same seizure; * unknown compound often coeluted with β-pinene (EI-mass spectrum: *43 55 67 79 95 (109) 111 119 123 137 152);* ** unknow compound (EI-mass spectrum: *41 55 67 79 91 105 119 133 147 161 175 (189) 204);**** β-calacorene often coeltued with Selina-4(15),7(11)-diene.

In short, chemical investigations performed on different samples of *Cannabis sativa* L. (hashish, fresh and dried buds) shed light on the occurrence of a rare monoterpene, *i*.*e*. compound 1, among the main volatiles constituents of hashish. Accordingly, compound 1 may provide a useful marker for the detection, such as remote-sensing, of hashish (resin) and marijuana (dry buds) in a sample for the following reasons: 1/ compound 1 is very rare among natural constituents of essential oils and other raw materials usually issued from the plant kingdom. A total of 6 bibliographic references related to compound 1 are found with the help of SciFinder (CAS online), and only one actually deals with the identification of this compound in a natural extract in trace levels (< 0.1 %); 2/ analyses performed on the different samples of hashish demonstrate compound 1 as being among the most abundant constituents in the sample headspace, even at room temperature. Compound 1 may thus be considered as specific marker of hashish. Although compound 1 has been detected in cannabis herbs (i.e. marijuana) in lower amount compared to hashish samples (< 0.5 % in the headspace), Compound 1 may also be considered as marker of marijuana. These findings may have a profound impact on the knowledge of VOCs emitted by drugs, and fulfill the actual needs of new markers, methods and devices for illegal drugs (remote) detection in the frame of drug regulation and law enforcement in Europe and other countries worldwide.

Although the above-mentioned embodiments have been described in detail, it is understood that alternative embodiments of the disclosure may be envisaged. Thus, for example, separation techniques other than solvent extraction or HS-SPME may be used to provide the method according to the present disclosure. In the same manner, analytic methods other than GC and GC-MS may be used to identify compound 1 according to the method of the present disclosure.

## Claims

1. A method for detecting the presence of hashish in a sample, the method comprising:
detecting a presence of 5,5-dimethyl-1-vinylbicyclo[2.1.1]hexane (1) in the sample.

2. The method of claim 1, further comprising:
separating constituents from a sample,
subjecting the constituents to an analytical method, and
identifying 5,5-dimethyl-1-vinylbicyclo[2.1.1]hexane (1) among the constituents.

3. The method of claim 1 or claim 2, wherein the presence of hashish in the sample is detected if 5,5-dimethyl-1-vinylbicyclo[2.1.1]hexane (1) amounts for at least 0.02 weight percent of the sample.

4. The method of claim 2 or claim 3, wherein the constituents are separated from the sample by solid phase microextraction (SPME).

5. The method of any of claims 2 to 4, wherein the analytical method is selected from the group comprising a chromatography technique, mass spectrometry (MS), infrared (IR), RAMAN, UV/UV-visible spectroscopies, nuclear magnetic resonance (NMR), ion-mobility spectrometry (IMS), and any combination thereof.

6. The method of claim 5, wherein the chromatographic technique is based on gas chromatography (GC).

7. The method of any of claims 2 to 6, wherein the analytical method comprises a gas chromatograph (GC) coupled to a mass spectrometer (MS), and/or an infrared (IR) spectrometer, and/or an ion-mobility spectrometer (IMS), and any combination thereof.

8. The method of any of claims 5 to 7, wherein the analytical method comprises an enantioselective analytical technique.

9. The method of claim 8, wherein 5,5-dimethyl-1-vinylbicyclo[2.1.1]hexane (1) is identified as a racemate.

10. Use of 5,5-dimethyl-1-vinylbicyclo[2.1.1]hexane (1) as marker of the presence of hashish in a sample.

11. A device for detecting the presence of hashish in a sample, the device comprising:
means for detecting a presence of 5,5-dimethyl-1-vinylbicyclo[2.1.1]hexane (1) in the sample.

12. The device according to claim 11, wherein the means for detecting comprises:
means for separating constituents from the sample, and
means for identifying 5,5-dimethyl-1-vinylbicyclo[2.1.1]hexane (1) among the constituents.

13. The device according to claim 11 or claim 12, wherein the means for separating compound 1 from the sample comprises one selected from the group comprising a gas chromatography system, a liquid chromatography system, a sorbent extraction tool, a trapping module, and any combination thereof.

14. The device according to claim 13, wherein the sorbent extraction tool comprises a solid phase microextraction (SPME) kit, a gas-tight syringe, and/or a tenax® adsorbent, and/or wherein the trapping module comprises molecular captands and/or sensors.

15. The device according to any of claims 11 to 14, wherein the means for identifying comprises a system selected from the group comprising gas chromatography (GC), liquid chromatography (LC), mass spectrometry (MS), infrared (IR), RAMAN, and UV/UV-visible spectroscopies, nuclear magnetic resonance (NMR), ion-mobility spectrometry (IMS), and any combination thereof.

16. The device according to any of claims 11 to 15, wherein the means for identifying comprises a gas chromatograph (GC) coupled to a mass spectrometer (MS), and/or an infrared (IR) spectrometer, and/or an ion-mobility spectrometer (IMS), and any combination thereof.
